(19) Europäisches Patentamt
European Patent Office
Office européen des brevets

(11) Publication number: 0 058 046
B1

(12) # EUROPEAN PATENT SPECIFICATION

(45) Date of publication of the patent specification: **01.08.84**

(51) Int. Cl.³: **C 07 C 47/22**, C 07 C 45/32, C 07 C 51/21, C 07 C 57/05, B 01 J 23/88, B 01 J 23/89

(21) Application number: **82300541.8**

(22) Date of filing: **03.02.82**

(54) **Process for producing methacrolein and methacrylic acid.**

(30) Priority: **05.02.81 JP 14944/81**
**17.02.81 JP 20895/81**

(43) Date of publication of application: **18.08.82 Bulletin 82/33**

(45) Publication of the grant of the patent: **01.08.84 Bulletin 84/31**

(84) Designated Contracting States: **DE FR GB**

(56) References cited:
**DE - B - 2 427 670**
**GB - A - 2 023 597**
**GB - A - 2 045 635**
**GB - A - 2 046 252**
**US - A - 4 155 938**

(73) Proprietor: **Ube Industries, Ltd., 12-32, Nishihonmachi 1-chome, Ube-shi, Yamaguchi-ken (JP)**

(72) Inventor: **Ohdan, Kyoji c/o Chuokenkyosho, UBE INDUSTRIES LTD. 1978-5, Oaza Kogushi, Ube-shi Yamaguchi-ken (JP)**
Inventor: **Suzuki, Kenichi c/o Chuokenkyusho, UBE INDUSTRIES LTD. 1978-5, Oaza Kogushi, Ube-shi Yamaguchi-ken (JP)**
Inventor: **Yamao, Takeshi c/o Chuokenkyusho, UBE INDUSTRIES LTD. 1978-5, Oaza Kogushi, Ube-shi Yamaguchi-ken (JP)**

(74) Representative: **George, Roger David et al, Raworth, Moss & Cook 36 Sydenham Road, Croydon Surrey CR0 2EF (GB)**

EP 0 058 046 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

1. The present invention relates to a process for producing methacrolein and methacrylic acid by the catalytic oxidation of tert.-butyl alcohol in a vapor phase with molecular oxygen at an elevated temperature. More particularly, the present invention relates to a process for producing methacrolein in a high yield together with methacrylic acid by the catalytic oxidation of tert.-butyl alcohol in a vapor phase with molecular oxygen at an elevated temperature by using a specific type of catalyst having a more enhanced catalytic activity, durability and mechanical strength than those of conventional catalysts containing molybdenum, bismuth, iron, cobalt, cesium and other specific elements as catalytic ingredients.

*Background of the invention*

Known are various types of processes and catalysts for producing methacrolein and methacrylic acid by the catalytic oxidation of tert.-butyl alcohol in a vapor phase with molecular oxygen at an elevated temperature.

For example, Japanese Patent Application Laid-open Nos. 50-105606 (1975), 50-96512 (1975), 51-113807 (1976), 55-85535 (1980), 55-62035 (1980) and other certain publications disclose various types of catalysts effective for the above-mentioned processes. These catalysts contain, as indispensable catalytic ingredients, molybdenum, bismuth, iron, cobalt and a plurality of other specific elements, for example, antimony and/or tungsten. When these catalysts are used, however, the yield of methacrolein and the sum of the yields of methacrolein and methacrylic acid are usually not satisfactory and the mechanical strength and durability of the catalysts are also not satisfactory from the viewpoint of industrial use.

The United Kingdom Patent Application No. 2023597A describes a process in which methacrolein is produced in a high yield together with a small amount of methacrylic acid by catalytically oxidizing tert.-butyl alcohol at a temperature of from 250 to 500°C by using a catalyst comprising an oxide composition of the empirical formula:

$$Mo_aCo_bFe_cBi_dCs_eX_fY_gO_h$$

wherein X is either or both of vanadium and palladium, Y is at least one member selected from titanium, tin and zirconium, a=12, b=2-12, c=0.5-7, d=0.1-5, e=0.0005-0.5, f=0.01-2, g=0-5 and h is a positive number proportional to the number of oxygen atoms satisfying the average valency of the metal atoms stated in the formula, which catalyst has a high resistance to compression and attrition and exhibits a high catalytic efficiency, mechanical strength and durability.

West German Patent Application No. 2427670, equivalent to U.K. Patent No. 1444659, describes a process for producing methacrolein from isobutylene or t-butyl alcohol, or methacrolein and 1,3-butadiene by simultaneously oxidising isobutylene and n-butene, using a catalyst comprising oxides of Mo, Sb, Bi, Fe, Ni, Sn, K, Rb and/or Cs; and Pd, Co, V, Ge, W and/or Ti. The catalyst contains Sb and Ni as essential constituents.

Generally speaking, it is known that, in catalytic reactions, the catalytic effects of the catalyst used are significantly variable, depending on the types of catalytic ingredients and on the composition of the catalyst. Therefore, it is extremely difficult to make an accurate estimate of the catalytic effects of a new catalyst from the composition of the catalyst.

Under these circumstances, it is desired to provide a catalytic process which enables methacrolein and methacrylic acid to be concurrently produced in a high yield and which exhibits an enhanced mechanical strength and durability.

As a result of a study on the improvement of the catalyst it was discovered by the inventors of the present invention that the catalytic efficiency and the mechanical strength and durability of the catalyst can be enhanced by using cesium as an alkali metal catalytic ingredient and by adding an additional catalytic ingredient consisting of at least one member selected from the group of vanadium and palladium, and, optionally, a further additional catalytic ingredient consisting of at least one member selected from the group consisting of titanium, tin and zirconium to the conventional catalytic ingredients, that is, molybdenum, cobalt, iron and bismuth.

*Summary of the invention*

An object of the present invention is to provide a process for producing methacrolein and methacrylic acid by the catalytic oxidation of tert.-butyl alcohol in a vapor phase with molecular oxygen at an elevated temperature in the presence of a catalyst which is capable of enhancing the yield of methacrolein and which has an enhanced mechanical strength and durability.

The above-mentioned object can be attained by the process of the present invention which comprises bringing a feed gas, containing tert.-butyl alcohol and molecular oxygen, into contact with a catalyst at an elevated temperature, which process is characterized in that the catalyst comprises an oxide composition of the empirical formula (I):

$$Mo_aCo_bFe_cBi_dCs_eX_fY_gO_h \quad (I)$$

wherein Mo represents a molybdenum atom, Co represents a cobalt atom, Fe represents an iron atom, Bi represents a bismuth atom, Cs represents a cesium atom, X represents at least one member selected from the group consisting of vanadium and palladium atoms, Y represents at least one member selected from the group consisting of titanium, tin and zirconium atoms, O represents an oxygen atom, the subscripts a through g respectively represent a positive number proportional to the number of respective metal atoms, wherein when a is 12, b through g fall respectively within the following ranges: b=2 to 12, c=0.5 to 7, d=0.1 to 5, e=0.0005 to 0.5, f=0.01 to 2 and g=0 to 5,

and the subscript h represents a positive number corresponding to the number of oxygen atoms satisfying the average valence of the above-mentioned metal atoms. It is preferable that the values of the subscripts b through g be respectively in the ranges of: b=4 to 10, c=1 to 5, d=0.5 to 4, e=0.001 to 0.3, f=0.05 to 1.5 and g=0.1 to 5.

*Detailed description of the invention*

In the catalyst usable for the process of the present invention, it is important that cesium is used as an alkali metal catalytic ingredient, either or both of vanadium and palladium are added, as an additional catalytic ingredient, to the conventional catalytic ingredient, that is, molybdenum, bismuth, iron, cobalt and an alakli metal, and optionally, that a further additional catalytic ingredient consisting of one, two, or more members from the group consisting of titanium, tin and zirconium is added to the above-mentioned conventional and additional catalytic ingredients. If an alkali metal, such as potassium, sodium, rubidium and lithium, which are different from cesium, is used, the resultant catalyst will result in a poor yield of methacrolein and in a poor sum of yields of methacrolein and methacrylic acid. Accordingly, the alkali metal which are different from cesium cannot be used to attain the object of the present invention. Even if cesium is used as an alkali metal catalytic ingredient, if the additional catalytic ingredient consisting of at least one member selected from vanadium and palladium is not used, the resultant catalyst will result in a poor yield of methacrolein. Also, in the case where cesium is used and the additional catalytic ingredient consisting of vanadium and/or palladium is replaced by at least one member selected from P, As, B, W, Sb, Te, Cr, Ti, Sn, Zr, Zn, Cu, Al and Se in an amount of 0.5 atoms per atom of molybdenum, the resultant catalyst results in an unsatisfactory yield of methacrolein.

Furthermore, even if the additional catalytic ingredient is used, if no cesium is used, the use of the resultant catalyst will result in a poor yield of methacrolein.

That is, the catalyst containing cesium and vanadium and/or palladium is effective for producing methacrolein in an increased yield thereof. However, with respect to this type of catalyst, it is desirable to enhance resistances thereof to attrition and compression. Also, it is desirable to additionally increase the yield of methacrolein.

It was discovered by the inventors of the present invention that, in the catalytical production of methacrolein and methacrylic acid, the addition of the further additional catalytic ingredient, consisting of at least one member selected from the group comprising titanium, tin and zirconium, is not only effective for enhancing the mechanical strength and durability of the resultant catalyst, but also, contributive for increasing the yield of methacrolein.

If the further additional catalytic ingredient is replaced by at least one element selected from P, As, B, W, Sb, Te, Cr, Zn, Cu, Al and Se, the contribution of the elements on the yield of methacrolein and the resistances to attrition and compression rupture, is very small and unsatisfactory.

The further additional catalytic ingredients, namely, titanium, tin and zirconium, may be used either singly or in combination of two or more thereof. However, the use of a combination of two or more ingredients is not remarkably superior to the use of a single ingredient in the yield of methacrolein, in the sum of yields of methacrolein and methacrylic acid and in the resistances of the resultant catalyst to attrition and compression.

The catalyst usable for the present invention must have the empirical composition specified hereinbefore. If the composition of the catalyst falls outside of the specified range of the composition, the resultant catalyst will result in an unsatisfactory yield of methacrolein and in the sum of yields of methacrolein and methacrylic acid. As long as the composition of the catalyst falls within the range specified above, not only is it possible for the process of the present invention to produce methacrolein and methacrylic acid, especially, methacrolein, in an extremely high yield thereof, but, also, the resultant catalyst exhibits enhanced resistance to attrition and crush strength. Therefore, the process of the present invention can be stably carried out over a long period of time without disintegration of the catalyst and change in the catalytic efficiency of the catalyst. Accordingly, it is obvious that the process of the present invention can be very advantageous for continuously producing methacrolein and methacrylic acid on an industrial scale.

In the catalyst usable for the present invention, the respective catalytic ingredients are present in the form of oxides, which include those of the type in which each single element is bonded with oxygen, those of the type in which two or more elements are bonded with oxygen to form a complex and those of the type in which the above-mentioned two types of oxides are combined together.

The catalyst of the above empirical formula (I), usable for the process of the present invention, may be prepared in any conventional manner by using, as the starting raw material, oxides, salts and other compounds, containing the above-mentioned catalytic ingredients. However, calcination of the catalyst, i.e. the final step of the catalyst preparation, should preferably be carried out at a temperature in the range of from 550° C to 800° C, more preferably, from 600° C to 750° C, and over a period of 1 to 20 h, more preferably from 2 to 10 h, for obtaining the desired yield of methacrolein and catalyst strength. This temperature range is higher than the range of from 400 to 500° C usually employed for preparing the conventional catalysts based on molybdenum, bismuth, iron and cobalt.

A calcining temperature falling outside of the above-mentioned range may sometimes cause a decrease in the mechanical strength of the

resultant catalyst and/or the yield of methacrolein. The calcining operation is carried out in a gas containing oxygen, usually an air atmosphere.

The general procedures for preparing a catalyst are as follows. Oxides, salts and other compounds containing the above-mentioned metal ingredients are mixed together in an aqueous medium to prepare a uniform slurry. The aqueous slurry is dried at a temperature not exceeding 300° C. In this case, the drying operation is preferably carried out in two steps. That is, in the first step, the aqueous dispersion is heated at a temperature of from 100 to 150° C, preferably at approximately 120° C, to evaporate water; and in the second step, the dried product is heated at a temperature, higher than that in the first step, of from 150 to 300° C, preferably at approximately 200° C, for a time period of 3 to 20 h to eliminate volatile substances, for example, ammonium nitrate and nitrogen oxides. The dried product is shaped into pellets or particles of a desired shape and size. The shaped pellets or particles are calcined under the above-mentioned conditions.

As examples of the starting raw materials which can be used in the preparation of the catalyst are, for example, molybdenum compounds, such as molybdic acid, ammonium molybdate and molybdenum trioxide; cobalt compounds, such as cobalt carbonate, cobalt nitrate, cobaltous oxide, tricobalt tetroxide, cobalt chloride, cobaltous hydroxide and cobaltic hydroxide; iron compounds, such as ferrous nitrate, ferric nitrate, ferrous oxide, ferric oxide, ferrous carbonate, ferrous chloride, ferric chloride, ferrous hydroxide and ferric hydroxide; bismuth compounds, such as bismuth nitrate, bismuth dichloride, bismuth trichloride, bismuth pentoxide, bismuth trioxide, bismuth tetroxide, bismuth oxynitrate, bismuth hydroxide, bismuth subnitrate and bismuth oxychloride; cesium compounds, such as cesium nitrate, cesium chloride, cesium hydroxide, cesium carbonate and cesium oxide; vanadium compounds, such as vanadium chloride, ammonium metavanadate, vanadyl chloride, and vanadium pentoxide; palladium compounds, such as palladium nitrate, palladium hydroxide, palladium chloride and palladium oxide; titanium compounds, such as titanium dioxide, titanic acid, titanium trichloride and titanium tetrachloride; thin compounds, such as stannic oxide, tin hydroxide, stannic chloride, stannous oxide and stannic acid; and zirconium compounds, such as zirconium oxide, zirconium oxynitrate and zirconium hydroxide. Among the above-mentioned compounds, the compounds which are most preferable as starting raw materials are nitrates and ammonium salts of the respective ingredient metals.

The procedures for preparing a catalyst usable for the present invention will be described in more detail with reference to a catalyst consisting of molybdenum, cobalt, iron, bismuth, cesium, vanadium and oxygen.

Predetermined amounts of ammonium molybdate and ammonium metavanadate are dissolved in water, preferably warm water, to prepare an aqueous solution. Separately, solution of a predetermined amount of bismuth nitrate in nitric acid and predetermined amounts of ferric nitrate, cobalt nitrate and cesium nitrate are dissolved in water, preferably warm water. This acidic solution of the nitrates is mixed dropwise with the above-mentioned solution of molybdenum and vanadium, while the mixture is being stirred. The so-obtained aqueous slurry is then heated at a temperature of from 100 to 150° C, preferably at approximately 120° C, by using a drum dryer or a spray dryer to evaporate water from the slurry. Next, the evaporation residue is heated at a temperature of from 150 to 300° C, preferably at approximately 200° C, until no more ammonium nitrate and nitrogen oxides are evolved from the dried product and the residue is completely dried. The resultant dried product is shaped or granulated into a desired shape and size of pellets or particles. If necessary, the particles can be separated by using a screen having a desired mesh to collect particles having a particular desired size. The particles are finally calcined, preferably at a temperature of from 550 to 800° C, more preferably from 600 to 750° C.

In the case where palladium is used in place of vanadium as a catalytic metal ingredient, it is preferable to use palladium nitrate as a starting raw material. A predetermined amount of palladium nitrate is dissolved together with ferric nitrate, cobalt nitrate and cesium nitrate in warm water. The solution is added dropwise into a solution of ammonium molybdate alone in warm water while the resultant mixture is being stirred.

In the case where the catalyst contains a further additional catalytic ingredient, for example titanium, it is preferable that titanium dioxide be suspended in an aqueous solution of ammonium molybdate alone or together with ammonium metavanadate. Also, in the case where the catalyst contains a further additional catalytic ingredient, it is preferable to carry out the calcining operation at a temperature of from 550 to 800° C, more preferably from 600 to 750° C in an oxygen-containing atmosphere.

The procedures and starting materials of the catalyst usable for the present invention are not limited to the above-mentioned examples. However, it is important that the starting materials be mixed with each other in the presence of water, the mixture be dried in two separate steps and then the dried mixture be calcined at a temperature of from 550 to 800° C, preferably from 660 to 750° C. This feature is effective for increasing the reproductivity in the catalytic activity and the durability and attrition resistance of the catalyst.

The catalyst may be used alone or in combination with a carrier. Carriers, such as those known for supporting conventional oxidation catalysts and for bringing about favorable effects to the reaction involved, e.g. silica, alumina, silica-alumina, titania, diatomaceous earth and carborundum, may be used. These carriers may be combined with the catalyst either during or after preparation of the catalyst.

In general, the size and shape of the catalyst particle used, and the use of a carrier are not critical factors, because they do not greatly affect the resultant catalytic activity.

In carrying out the process of the present invention, an inert gas, which is not reactive to catalytic oxidation, may be used as a diluent gas for the feed gas. The diluent gas may be steam, nitrogen gas, carbon dioxine gas, n-butane gas, isobutane gas or propane gas. Especially, the addition of steam as the inert gas to the feed gas is effective for increasing the yield of methacrolein and the durability in catalytic activity of the catalyst. Therefore, it is preferable that the catalytic oxidation be carried out in the presence of steam. The steam is used preferably in an amount of from 0.05 to 10 mol, more preferably from 0.1 to 8 mol, per mole of tert.-butyl alcohol.

In the catalytic oxidation of the present invention, the molecular oxygen used need not be highly purified. That is, oxygen-containing gases, such as air or a mixture of pure molecular oxygen and the above-mentioned diluent gas, may be conveniently used. Air, particularly, may be advantageously used. The relative proportion of molecular oxygen in the feed gas is usually in the range of from 0.4 to 5 mol, more preferably from 0.5 to 3 mol, per mole of tert.-butyl alcohol.

In the catalytic oxidation of the present invention the contact of the feed gas with the catalyst is carried out preferably at a temperature in the range of from 250 to 500° C, more preferably from 300 to 40° C. The contact time is usually in the range of from 0.3 to 20 s, preferably from 0.5 to 15 s. The reaction may be carried out under atmospheric pressure, although superatmospheric or subatmospheric pressure may be used if desired. However, atmospheric pressure is preferable.

The catalytic oxidation reaction may be carried out in a fixed bed, a moving bed or a fluidized bed.

If it is necessary, the resultant methacrolein and methacrylic acid can be isolated from the reaction mixture by using any kind of conventional isolating processes, for example by the absorption of methacrolein and methacrylic acid by cold water followed by the stripping and distillation of methacrolein and methacrylic acid.

The present invention will be further clarified by the examples and comparison examples set forth below. In all of the examples "%" is expressed by weight unless otherwise specified. In these examples, the conversion of tert.-butyl alcohol and yield of methacrolein or methacrylic acid were calculated in accordance with the following equations.

Percent conversion of tert.-butyl alcohol =

$$\frac{\text{moles of tert.-butyl alcohol consumed}}{\text{moles of tert.-butyl alcohol fed}} \times 100$$

Percent yield of methacrolein or methacrylic acid =

$$\frac{\text{moles of methacrolein or methacrylic acid produced}}{\text{moles of tert.-butyl alcohol fed}} \times 100$$

The moles of tert.-butyl alcohol fed, the moles of tert.-butyl alcohol consumed and the moles of methacrolein or methacrylic acid produced were determined after one hour had elapsed from the start of the reaction.

The crush strength of the catalyst was determined as follows.

A catalyst tablet (5 mm in diameter and 5 mm in height) was placed on a testing plate and compressed by using a Kiya-type hardness tester until the tablet was crushed. The value of the compressive load in kilogram under which the tablet was crushed was measured. The above-mentioned testing procedures were applied to 50 tablets. The rush strength of the catalyst tablet was represented by an average value calculated from the results of 50 tablets.

The resistance of the catalyst to attrition was determined as follows.

A glass test tube having an inner diameter of 2.54 cm and a height of 300 cm was used. 50 tablets of a catalyst were dropped down from the top of the glass test tube to the bottom thereof. The total weight of the tablets which were crushed into particles having a mesh size of 6 or less was measured. The resistance of the catalyst to attrition was thereafter represented by a ratio (%) of the weight of the crushed tablets to the weight of the original 50 tablets.

*Example 1:*

In order to provide an aqueous solution suspension, 141.3 g of ammonium molybdate $[(NH_4)_6Mo_7O_{24} \cdot 4H_2O]$, and 0.78 g of ammonium metavanadate $[NH_4VO_3]$ were dissolved in 200 ml of water maintained at a temperature of 40° C. A solution of 38.8 g of bismuth nitrate $[Bi(NO_3)_3 \cdot 5H_2O]$ in 50 ml of a 15% nitric acid aqueous solution was mixed into a solution of 64.6 g of ferric nitrate $[Fe(NO_3)_3 \cdot 9H_2O]$, 0.078 g of cesium nitrate $[CsNO_3]$ and 186.2 g of cobalt nitrate $[Co(NO_3)_2 \cdot 6H_2O]$ in 200 ml of water having a temperature adjusted to 40° C. The mixture was admixed dropwise into the above-prepared aqueous solution of ammonium molybdate and ammonium metavanadate while the admixed solution was being stirred.

The admixed solution was first dried at a temperature of 120° C by using a drum dryer. The initially dried material was additionally dried at a temperature of 200° C for 10 h by using an oven. The resultant material was shaped into tablets having a diameter of 5 mm and a length of 5 mm by means of a tablet-forming machine. The tablets were calcined at a temperautre of 650° C for 5 h in an air atmosphere to provide a catalyst. In the catalyst thus prepared, the atomic ratio of Mo:Bi:Co:-Fe:Cs:V was 12:1:8:2:0.005:0.1.

10 ml of the catalyst tablets were charged into a U-shaped glass tube having an inner diameter of 8 mm. A feed gas containing tert.-butyl alcohol, air and steam in a molar ratio of 1:10:6 was passed through the U-shaped tube at a temperature of 390° C at a flow rate of 180 ml/min so that the contact time of the feed gas with the catalyst tablets was 3.3 s. The percent conversion of tert.-butyl alcohol, and the yields of methacrolein and methacrylic acid were determined. The results are shown in Table 1.

*Examples 2 through 6:*

In each of Examples 2 through 6, the same catalyst-preparing procedures as those described in Example 1 were carried out, except that the atomic ratio of Mo:Bi:Co:Fe:Cs:V was varied for each of the Examples 2 through 6 in accordance with the ratios shown in Table 1. The same catalytic oxidation procedures as those described in Example 1 were carried out, except that in Example 3, the catalytic oxidation was carried out at a temperature of 380° C. The results are shown in Table 1.

*Examples 7 and 8:*

In each of Examples 7 and 8, procedures identical to those described in Example 1 were carried out, except that 1.84 g of palladium nitrate [$Pd(NO_3)_2$] were added to the mixed solution. The atomic ratio of Mo:Bi:Co:Fe:Cs:V:Pd for each of Examples 7 and 8 is respectively shown in Table 1. Also, in Example 7, the catalytic oxidation was carried out at a temperature of 380° C. The results are shown in Table 1.

*Examples 9 and 10:*

In each of Examples 9 and 10, the same procedures as those used in Example 8 were carried out, except that no ammonium metavanadate was used. The atomic ratio of Mo:Bi:Co:Fe:Cs:Pd for each of the two examples is respectively shown in Table 1. In Example 9, the reaction temperature (catalytic contacting temperature) was 360° C. The results of Examples 9 and 10 are shown in Table 1.

*Table 1 ⟶*

*Table 1*

| Example No. | Composition of catalyst (Atomic ratio) | | | | | | | Reaction temperature (° C) | Conversion of tert.-butyl alcohol (%) | Yield of methacrolein (%) | Yield of methacrylic acid (%) | Sum of (*), yields (%) |
|---|---|---|---|---|---|---|---|---|---|---|---|---|
| | Mo | Co | Fe | Bi | Cs | V | Pd | | | | | |
| 1 | 12 | 8 | 2 | 1 | 0.005 | 0.1 | — | 390 | 100 | 85.2 | 2.3 | 87.5 |
| 2 | 10 | 8 | 2 | 1 | 0.05 | 0.1 | — | 390 | 100 | 84.3 | 2.1 | 86.4 |
| 3 | 10 | 8 | 2 | 1 | 0.1 | 0.5 | — | 380 | 100 | 83.0 | 2.0 | 85.0 |
| 4 | 12 | 7 | 4 | 1 | 0.01 | 0.1 | — | 390 | 100 | 84.8 | 2.1 | 86.9 |
| 5 | 12 | 8 | 3 | 2 | 0.01 | 0.1 | — | 390 | 100 | 85.1 | 2.2 | 87.3 |
| 6 | 11 | 6 | 4 | 1 | 0.01 | 0.1 | — | 390 | 100 | 84.9 | 2.0 | 86.9 |
| 7 | 10 | 8 | 2 | 1 | 0.05 | 0.1 | 0.1 | 380 | 100 | 85.0 | 2.3 | 87.3 |
| 8 | 11 | 8 | 3 | 1 | 0.01 | 0.1 | 0.1 | 390 | 100 | 84.7 | 2.1 | 86.8 |
| 9 | 10 | 8 | 2 | 1 | 0.005 | — | 0.1 | 360 | 100 | 84.6 | 2.2 | 86.8 |
| 10 | 10 | 8 | 2 | 1 | 0.1 | — | 0.5 | 390 | 100 | 85.6 | 2.0 | 87.6 |

Note: (*), — Sum of yield of methacrolein and yield of methacrylic acid.

*Comparison Example 1:*

The same procedures as those described in Example 1 were carried out, except that neither cesium nitrate nor ammonium metavanadate were used, the atomic ratio of the catalytic ingredients was the same as that shown in Table 2 and the reaction temperature was 370° C. The results are shown in Table 2.

*Comparison Examples 2 and 3:*

In each of Examples 2 and 3, the same procedures as those described in Example 1 were carried out, except that no ammonium metavanadate was used and the atomic ratio of the catalytic ingredients in the catalyst for each of the two Comparison Examples was the same as that respectively shown in Table 2. The results are shown in Table 2.

*Comparison Examples 4 through 7:*

In each of Examples 4 through 7, the same procedures as those described in Example 1 were carried out, except no ammonium metavanadate was used, cesium nitrate which served as the raw material for the alkali metal catalytic ingredient was replaced respectively by sodium nitrate in Comparison Example 4, by rubidium nitrate in Comparison Example 5 and by potassium nitrate in Comparison Examples 6 and 7 in accordance with the amounts respectively shown in Table 2, and the atomic ratio of the catalytic ingredients in the resultant catalyst for each of these Comparison Examples was the same as that respectively shown in Table 2. In Comparison Examples 4 and 7, catalytic oxidation was carried out at a temperature of 370° C. The results are shown in Table 2.

*Comparison Example 8:*

The same procedures as those described in Example 1 were carried out, except that no cesium nitrate was used, the atomic ratio of the catalytic ingredients in the resultant catalyst was the same as that shown for this Comparison Example in Table 2, and catalytic oxidation was carried out at a temperature for a contact time as specified in Table 2 for this Comparison Example. The results are shown in Table 2.

*Comparison Example 9:*

A comparison catalyst having an atomic ratio of the catalytic ingredients as shown in Table 2 was prepared by following procedures similar to those used in Example 9, except that no cesium nitrate was used. The same reaction procedures as those described in Example 1 were carried out, except that the reaction temperature and the contact time followed were those shown in Table 2 for this Comparison Example 9. The results are shown in Table 2.

*Comparison Examples 10 through 12:*

In each of Comparison Examples 10 through 12, a comparison catalyst was prepared by following the same procedures as those described in Example 1, except that cesium nitrate was replaced respectively by potassium nitrate in Comparison Example 10, by sodium nitrate in Comparison Example 11 and by rubidium nitrate in Comparison Example 12. Also, a catalytic conversion of tert.-butyl alcohol was carried out by carrying out the same procedures as those described in Example 1, except that the above-prepared comparison catalyst was used, and the reaction temperature and the contact time followed were those respectively specified in Table 2 for Comparison Examples 10 through 12.

The comparison catalysts of Comparison Examples 11 and 12 were prepared by calcining at temperatures of 660 and 620° C, respectively.

The results are shown in Table 2.

*Table 2 ⟶*

*Table 2*

| Comparison Example No. | Composition of catalyst (Atomic ratio) Mo | Co | Fe | Bi | Alkali metal | V | Pd | Contact time (s) | Reaction temperature (°C) | Conversion of tert.-butyl alcohol (%) | Yield of methacrolein (%) | Yield of methacrylic acid (%) | Sum of yields (%) |
|---|---|---|---|---|---|---|---|---|---|---|---|---|---|
| 1 | 10 | 8 | 2 | 1 | — | — | — | 3.3 | 370 | 100 | 50.1 | 2.8 | 52.9 |
| 2 | 10 | 8 | 2 | 1 | Cs = 0.03 | — | — | 3.3 | 390 | 99.1 | 61.9 | 2.3 | 64.2 |
| 3 | 10 | 8 | 2 | 1 | Cs = 0.1 | — | — | 3.3 | 390 | 98.0 | 25.3 | 1.8 | 27.1 |
| 4 | 10 | 8 | 3 | 1 | Na = 0.5 | — | — | 3.3 | 370 | 100 | 65.8 | 2.0 | 67.8 |
| 5 | 10 | 8 | 3 | 1 | Rb = 0.05 | — | — | 3.3 | 390 | 100 | 67.3 | 2.3 | 69.6 |
| 6 | 10 | 8 | 2 | 1 | K = 0.01 | — | — | 3.3 | 370 | 100 | 65.2 | 2.0 | 67.2 |
| 7 | 10 | 8 | 2 | 1 | K = 0.3 | — | — | 3.3 | 390 | 100 | 66.6 | 1.9 | 68.5 |
| 8 | 10 | 8 | 2 | 1 | — | 0.1 | 0.1 | 3.0 | 360 | 100 | 62.4 | 2.1 | 64.5 |
| 9 | 10 | 8 | 2 | 1 | — | — | — | 3.0 | 340 | 100 | 57.7 | 2.0 | 59.7 |
| 10 | 10 | 8 | 2 | 1 | K = 0.05 | 0.1 | — | 3.0 | 370 | 100 | 65.4 | 2.9 | 68.3 |
| 11 | 10 | 8 | 2 | 1 | Na = 0.1 | 0.1 | — | 3.0 | 370 | 100 | 67.1 | 2.0 | 69.1 |
| 12 | 10 | 8 | 2 | 1 | Rb = 0.1 | 0.1 | — | 3.0 | 390 | 99.9 | 65.8 | 2.7 | 68.5 |

*Comparison Examples 13 through 17*

In each of Comparison Examples 13 through 17, a comparison catalyst was prepared by following the same procedures as those described in Example 1, except that the amounts of the raw materials of the respective catalytic ingredients used were changed so that the resultant comparison catalysts had a catalytic ingredient composition, as shown in Table 3, which is outside of the scope of the present invention.

In addition, the calcining temperature was adjusted to 600° C in Comparison Examples 13 and 14, to 650° C in Comparison Example 15, to 570° C in Comparison Example 16 and to 660° C in Comparison Example 17.

By using the comparison catalysts, the same procedures for converting tert.-butyl alcohol as those described in Example 1 were carried out, except that the reaction temperature and the contact time were changed to those shown respectively in Table 3 for Comparison Examples 13 through 17.

The results are shown in Table 3.

*Comparison Example 18:*

A comparison catalyst was prepared in accordance with the same procedures as those used in Example 9, except that the cesium nitrate as a raw material of an alkali metal catalytic ingredient was replaced by potassium nitrate and the resultant catalyst had the composition described in Table 3. Next, the same procedures for catalytically converting tert.-butyl alcohol as those described in Example 1 were carried out, except that the reaction temperature and the contact time were changed to those shown in Table 3 for Comparison Example 18.

The results for this Comparison Example are shown in Table 3.

*Comparison Example 19:*

A comparison catalyst was prepared by following the same procedures as those used in Example 9, except that the amount of palladium nitrate employed was changed to a value which caused the composition of the resultant catalyst to be changed to that shown in Table 3 for Comparison Example 19, and the calcining temperature of 650° C was changed to 670° C. The resultant catalyst was used for carrying out the same procedures as those mentioned in Example 1 for catalytically converting tert.-butyl alcohol, except that the reaction temperature and the contact time were changed to those shown in Table 3 for Comparative Example 19.

The results for this Comparative Example are shown in Table 3.

*Table 3 →*

*Table 3*

| Comparison Example No. | Composition of catalyst (Atomic ratio) | | | | | | | Contact time (s) | Reaction temperature (° C) | Conversion of tert.-butyl alcohol (%) | Yield of methacrolein (%) | Yield of methacrylic acid (%) | Sum of yields (%) |
|---|---|---|---|---|---|---|---|---|---|---|---|---|---|
| | Mo | Co | Fe | Bi | Alkali metal | V | Pd | | | | | | |
| 13 | 12 | 1 | 6 | 1 | Cs = 0.01 | 0.1 | — | 3.3 | 390 | 100 | 66.2 | 2.4 | 68.6 |
| 14 | 13 | 8 | 0.5 | 1 | Cs = 0.01 | 0.1 | — | 3.3 | 390 | 100 | 65.1 | 2.2 | 67.3 |
| 15 | 12 | 8 | 3 | 0.05 | Cs = 0.01 | 0.1 | — | 3.0 | 390 | 100 | 63.4 | 2.9 | 66.3 |
| 16 | 11 | 8 | 2 | 1 | Cs = 0.75 | 0.1 | — | 3.3 | 410 | 98.3 | 29.7 | 1.7 | 31.4 |
| 17 | 12 | 8 | 3 | 1 | Cs = 0.01 | 3 | — | 3.0 | 370 | 100 | 53.8 | 2.0 | 55.8 |
| 18 | 10 | 8 | 2 | 1 | K = 0.05 | — | 0.1 | 3.0 | 370 | 100 | 61.5 | 2.3 | 63.8 |
| 19 | 12 | 8 | 3 | 1 | Cs = 0.01 | — | 3 | 3.0 | 360 | 100 | 51.2 | 2.1 | 53.3 |

*Comparison Examples 20 through 33:*

In each of the Comparison Examples 20 through 33, the same catalyst-preparing procedures as those described in Example 1 were carried out, except that ammonium metavanadate was replaced by phosphoric acid, arsenic pentaoxide, boric acid, ammonium paratungstate, antimony trioxide, tellurium, oxide, chromium nitrate, titanium dioxide, stannic oxide, zirconium oxide, zinc nitrate, cupric nitrate, aluminium nitrate or selenium dioxide, as indicated in Table 4. By using this catalyst, the same catalytic oxidation procedures of tert.-butyl alcohol as those described in Example 1 were carried out, except that the reaction temperature was as indicated in Table 4.

The results are indicated in Table 4.

*(Table 4 on the following page)*

*Comparison Examples 34 through 38:*

The same catalyst-preparing procedures as those described in Example 1 were carried out, except that the atomic ratio Mo:Co:Fe:Bi:Cs:V was as indicated in Table 5 and phosphoric acid, arsenic pentaoxide, boric acid, ammonium paratungstate or cupric nitrate was added as a comparative further additional ingredient. Also, the same catalytic oxidation procedures of tert.-butyl alcohol as those described in Example 1 were carried out by using the above-mentioned catalyst, except that the flow rate of the feed gas was 200 ml/min and the contact time was 3 s. The results are indicated in Table 5.

*Table 4*

| Comparison Example No. | Composition of catalyst (Atomic ratio) Mo | Co | Fe | Bi | Cs | Additional ingredient | Reaction temperature (°C) | Conversion of tert.-butyl alcohol (%) | Yield of methacrolein (%) | Yield of methacrylic acid (%) | Sum of yields (%) |
|---|---|---|---|---|---|---|---|---|---|---|---|
| 20 | 12 | 8 | 2 | 1 | 0.1 | P = 0.5 | 400 | 99.7 | 68.3 | 2.0 | 70.3 |
| 21 | 10 | 8 | 2 | 1 | 0.1 | As = 0.5 | 400 | 99.9 | 70.0 | 2.1 | 72.1 |
| 22 | 10 | 8 | 2 | 1 | 0.1 | B = 0.5 | 400 | 100 | 66.1 | 1.9 | 68.0 |
| 23 | 12 | 8 | 2 | 1 | 0.1 | W = 0.5 | 380 | 100 | 68.5 | 2.6 | 71.1 |
| 24 | 12 | 8 | 2 | 1 | 0.1 | So = 0.5 | 390 | 100 | 71.9 | 2.1 | 74.0 |
| 25 | 12 | 8 | 2 | 1 | 0.1 | Te = 0.5 | 390 | 100 | 70.3 | 2.2 | 72.5 |
| 26 | 12 | 8 | 2 | 1 | 0.1 | Cr = 0.5 | 380 | 100 | 67.6 | 2.5 | 70.1 |
| 27 | 10 | 8 | 2 | 1 | 0.1 | Ti = 0.5 | 400 | 100 | 76.3 | 2.3 | 78.6 |
| 28 | 10 | 8 | 2 | 1 | 0.1 | Sn = 0.5 | 380 | 100 | 75.9 | 2.6 | 78.5 |
| 29 | 10 | 8 | 2 | 1 | 0.1 | Zr = 0.5 | 400 | 100 | 74.9 | 2.2 | 77.1 |
| 30 | 10 | 8 | 2 | 0.5 | 0.05 | Zn = 1 | 400 | 100 | 70.2 | 2.0 | 72.2 |
| 31 | 10 | 7 | 2 | 1 | 0.05 | Cu = 0.5 | 380 | 100 | 68.3 | 2.4 | 70.7 |
| 32 | 10 | 7 | 2 | 1 | 0.05 | Al = 1 | 370 | 100 | 62.1 | 2.9 | 65.0 |
| 33 | 10 | 7 | 2 | 1 | 0.05 | Se = 1 | 380 | 100 | 65.3 | 2.6 | 67.9 |

*Comparison Example 39:*

The same catalyst-preparing procedures as those described in Example 1 were carried out, except that palladium nitrate and arsenic pentaoxide were added each in amounts indicated in Table 5. The same catalytic oxidation of tert.-butyl alcohol as that described in Comparison Example 34 was carried out by using the above-mentioned catalyst. The results are indicated in Table 5.

*Comparison Examples 40 and 41:*

The same catalyst-preparing procedures as those described in Comparison Example 39 were carried out, except that ammonium metavanadate was replaced by palladium nitrate and arsenic pentaoxide was replaced by tellurium oxide or phosphoric acid so as to provide an atomic ratio of the ingredients in the resultant catalyst indicated in Table 5.

By using the resultant catalyst, the same catalytic oxidation procedures of tert.-butyl alcohol as those described in Comparison Example 34 were carried out, except that in Comparison Example 41, the reaction temperature was 390° C.

The results are indicated in Table 5.

*(Table 5 on the following page)*

*Example 11:*

The same procedures as those described in Example 1 for catalytically converting tert.-butyl alcohol into methacrolein and methacrylic acid were continuously carried out for 1000 h by using the same catalyst ($Mo_{12}Co_8Fe_2Bi_1Cs_{0.005}V_{0.1}$) as that mentioned in Example 1.

After 1000 h had elapsed from the start of the reaction, the percent conversion of tert.-butyl alcohol was 100%, the percent yield of methacrolein was 85.3% and the percent yield of methacrylic acid was 2.5%.

The average crush strength of 50 catalyst tablets was 5.1 kg before the start of the reaction, and 5.1 kg 1000 h after the start of the reaction. That is, it was confirmed that the crush strength of the catalyst tablets did not change at all, even though the tablets were continuously used for a long period of time.

*Example 12:*

By using the same catalyst

$$(Mo_{10}Co_8Fe_2Bi_1Cs_{0.1}Pd_{0.5})$$

as that mentioned in Example 10, the same contacting procedures as those described in Example 1 were continuously carried out for 1000 h. After 1000 h had elapsed from the start of carrying out the contacting procedures, the percent conversion of tert.-butyl alcohol was 100%, the percent yield of methacrolein was 85.5% and the percent yield of methacrylic acid was 2.2% which were similar to those described in Example 10.

*Example 13:*

In 200 ml of warm water maintained at a temperature of 40° C, 141.3 g of ammonium molybdate [$(NH_4)_6MO_7O_{24}\cdot 4H_2O$] and 0.78 g of ammonium metavanadate [$NH_4VO_3$] were dissolved and 6.39 g of titanium dioxide were suspended. A mixture of a solution of 38.8 g of bismuth nitrate [$Bi(NO_3)_3\cdot 5H_2O$] in 50 ml of a 15% nitric acid aqueous solutions and a solution of

Table 5

| Comparison Example No. | Composition of catalyst (Atomic ratio) | | | | | | | | Reaction temperature (° C) | Conversion of tert.-butyl alcohol (%) | Yield of methacrolein (%) | Yield of methacrylic acid (%) | Sum of yields (%) |
|---|---|---|---|---|---|---|---|---|---|---|---|---|---|
| | Mo | Co | Fe | Bi | Cs | V | Pd | Further additional ingredient | | | | | |
| 34 | 12 | 8 | 2 | 1 | 0.005 | 0.1 | — | P = 0.5 | 400 | 100 | 77.3 | 2.1 | 79.4 |
| 35 | 12 | 8 | 2 | 1 | 0.005 | 0.1 | — | As = 0.5 | 400 | 100 | 75.6 | 2.6 | 78.2 |
| 36 | 12 | 8 | 2 | 1 | 0.005 | 0.1 | — | B = 0.5 | 400 | 100 | 76.3 | 2.0 | 78.3 |
| 37 | 12 | 8 | 2 | 1 | 0.005 | 0.1 | — | W = 0.5 | 390 | 100 | 75.9 | 1.8 | 77.7 |
| 38 | 10 | 7 | 2 | 1 | 0.05 | 0.1 | — | Cu = 0.5 | 390 | 100 | 76.3 | 2.1 | 78.4 |
| 39 | 10 | 8 | 2 | 1 | 0.1 | 0.1 | 0.1 | As = 0.5 | 400 | 100 | 76.3 | 2.4 | 78.7 |
| 40 | 10 | 8 | 2 | 1 | 0.1 | — | 0.1 | Te = 0.5 | 400 | 100 | 77.6 | 2.1 | 79.7 |
| 41 | 10 | 8 | 2 | 1 | 0.1 | — | 0.1 | P = 0.5 | 390 | 100 | 77.2 | 1.9 | 79.1 |

64.6 g of ferric nitrate [$Fe(NO_3)_3 \cdot 9H_2O$], 0.078 g of cesium nitrate [$CsNO_3$] and 186.2 g of cobalt nitrate [$CO(NO_3)_2 \cdot 6H_2O$] in 200 ml of warm water maintained at a temperature of 40° C, was admixed dropwise into the thus-prepared aqueous solution suspension while the admixed solution was being stirred.

The admixed aqueous slurry was initially dried at a temperature of 120° C by using a drum dryer, and then the first dried product was dried for a second time at a temperature of 200° C for 10 h by using an oven. The second dried product was shaped into tablets each having a diameter of 5 mm and a length of 5 mm by using a tablet-forming machine. The resultant tablets were calcined in an air atmosphere at a temperature of 650° C for 5 h. The resultant catalyst had an atomic ratio of Mo:Bi:-Co:Fe:Cs:V:Ti = 12:1:8:2:0.005:0.1:1. The measured crush strength and resistance to attrition of the catalyst tablets are shown in Table 6. 10 ml of the catalyst tablets were placed in a U-shaped glass reaction tube having an inner diameter of 8 mm. A feed gas consisting of tert.-butyl alcohol, air and steam in a molar ratio of 1:10:6 was passed through the reaction tube at a flow rate of 200 ml/min under such a condition that the contact of the feed gas with the catalyst was maintained for 3.0 s at a temperature of 390° C.

The results of Example 13 are shown in Table 6.

*Examples 14 through 17:*

The same procedures as those described in Example 13 were carried out, except that the titanium dioxide was replaced by zirconium oxide in Example 14, by stannic oxide in Example 15, by a mixture of zirconium oxide and stannic oxide in Example 16 and by a mixture of titanium dioxide and stannic oxide in Example 17, respectively in the amounts shown in Table 6, and that, in only Example 15, the conversion of tert.-butyl alcohol was carried out at a temperature of 370° C. The obtained catalysts had the compositional make-ups, the crush strength and the resistance to attrition as shown in Table 6.

The results of the conversion of tert.-butyl alcohol in the respective Examples 14 through 17 are shown in Table 6.

*Example 18:*

Procedures identical to those described in Example 13 were carried out, except that no ammonium metavanadate was used and palladium nitrate was contained in the aqueous solution of ferric nitrate, cesium nitrate and cobalt nitrate. The resultant catalyst tablets had the compositional make-ups, the crush strength and the resistance to attrition as shown in Table 6.

Also, the results of the catalytic conversion of tert.-butyl alcohol are shown in Table 6.

*Examples 19 and 20:*

Procedures identical to those used in Example 18 were conducted, except that the titanium dioxide employed in Example 18 was replaced by zirconium oxide in Example 19, and by stannic oxide in Example 20 and that the resultant catalyst had the compositional make-ups, the crush strength and resistance to attrition as shown in Table 6. Also, the results of the catalytic conversion of tert.-butyl alcohol are shown in Table 6.

*Examples 21 and 22:*

Procedures identical to those described in Example 13 were carried out, except that in both Examples 21 and 22, palladium nitrate was added to the aqueous solution of ferric nitrate, cesium nitrate and cobalt nitrate and the conversion of tert.-butyl alcohol was conducted at a temperature of 370° C. Additionally, in Example 22, the titanium dioxide was replaced by stannic oxide. The catalyst tablets prepared had the compositional make-ups, the crush strength and the resistance to attrition as shown in Table 6.

The results of the conversions of tert.-butyl alcohol in both Examples 21 and 22 are also shown in Table 6.

*Comparison Example 42:*

The same procedures as those carried out in Example 22 were effected, except that no ammonium metavanadate and no stannic oxide were used.

The properties of the resultant catalyst and the results of the conversion of tert.-butyl alcohol in the presence of the catalyst are indicated in Table 6.

*Table 6 ⟶*

*Comparison Examples 43 through 48:*

In each of the Comparison Example 43 through 48, the same procedures as those described in Example 13 were carried out with the following exceptions.

(1) The resultant catalyst in each Comparison Example had the compositional make-up indicated in Table 7.

(2) In Comparison Example 44, the contacting temperature was 370° C.

The results are indicated in Table 7.

*(Table 7 on the following page)*

## Claims

1. A process for producing methacrolein and methacrylic acid by the catalytic oxidation of tert.-butyl alcohol, comprising bringing a feed gas containing tert.-butyl alcohol and molecular oxygen into contact with a catalyst at a elevated temperature, which process is characterized in that said catalyst comprises an oxide composition of the empirical formula (I):

$$Mo_aCo_bFe_cBi_dCs_eX_fY_gO_h \quad (I)$$

wherein Mo represents a molybdenum atom, Co represents a cobalt atom, Fe represents an iron atom, Bi represents a bismuth atom, Cs represents a cesium atom, X represents at least one member selected from the group consisting of vanadium and palladium atoms, Y represents at least one member selected from the group consisting of titanium, tin and zirconium atoms, O represents an oxygen atom, the subscripts a through g respectively represent a positive number corresponding to the number of respective metal atoms, wherein when a=12, b through g fall respectively within

*Table 6*

| Example No. | Composition of catalyst (Atomic ratio) Mo | Co | Fe | Bi | Cs | X | Y | Crush strength (kg/tablet) | Resistance to attrition (wt %) | Conversion of tert.-butyl alcohol (%) | Yield of methacrolein (%) | Yield of methacrylic acid (%) | Sum of yields (%) |
|---|---|---|---|---|---|---|---|---|---|---|---|---|---|
| 13 | 12 | 8 | 2 | 1 | 0.005 | V = 0.1 | Ti = 1 | 6.6 | 0.02 | 100 | 86.7 | 2.9 | 89.6 |
| 14 | 10 | 8 | 2 | 1 | 0.01 | V = 0.1 | Zr = 1 | 6.7 | 0.02 | 100 | 85.4 | 2.7 | 88.1 |
| 15 | 10 | 8 | 3 | 1 | 0.005 | V = 0.2 | Sn = 2 | 6.2 | 0.04 | 100 | 85.3 | 2.7 | 88.0 |
| 16 | 10 | 8 | 2 | 1 | 0.01 | V = 0.1 | Zr = 1<br>Sn = 1 | 6.5 | 0.03 | 100 | 86.5 | 2.4 | 88.9 |
| 17 | 10 | 8 | 2 | 1 | 0.01 | V = 0.2 | Ti = 1<br>Sn = 1 | 6.7 | 0.02 | 100 | 85.0 | 2.6 | 87.6 |
| 18 | 10 | 8 | 2 | 2 | 0.05 | Pd = 0.1 | Ti = 1 | 7.1 | 0.02 | 100 | 86.0 | 2.6 | 88.6 |
| 19 | 10 | 8 | 3 | 2 | 0.01 | Pd = 0.2 | Zr = 2 | 6.5 | 0.02 | 100 | 86.1 | 2.5 | 88.6 |
| 20 | 10 | 8 | 2 | 1 | 0.01 | Pd = 0.1 | Sn = 2 | 7.0 | 0.02 | 100 | 85.3 | 2.3 | 87.6 |
| 21 | 10 | 8 | 2 | 1 | 0.01 | V = 0.1<br>Pd = 0.1 | Ti = 1 | 6.0 | 0.02 | 100 | 85.2 | 2.8 | 88.0 |
| 22 | 10 | 8 | 2 | 0.5 | 0.01 | V = 0.1<br>Pd = 0.1 | Sn = 2 | 7.2 | 0.04 | 100 | 86.7 | 2.6 | 89.3 |
| Comparison | | | | | | | | | | | | | |
| 42 | 10 | 8 | 2 | 0.5 | 0.01 | Pd = 0.1 | 0 | 5.3 | 0.30 | 100 | 82.9 | 2.3 | 85.2 |

*Table 7*

| Comparison Example No. | Composition of catalyst (Atomic ratio) | | | | | | | Conversion of tert.-butyl alcohol (%) | Yield of methacrolein (%) | Yield of methacrylic acid (%) | Sum of yields (%) |
|---|---|---|---|---|---|---|---|---|---|---|---|
| | Mo | Co | Fe | Bi | Alkali metal | X | Y | | | | |
| 43 | 10 | 8 | 3 | 1 | 0 | 0 | Ti = 2 | 100 | 60.3 | 2.4 | 62.7 |
| 44 | 10 | 8 | 2 | 1 | 0 | V = 0.5 | Sn = 1 | 100 | 67.5 | 2.0 | 69.5 |
| 45 | 10 | 8 | 3 | 1 | 0 | Pd = 0.1 | Zr = 1 | 100 | 78.3 | 2.1 | 70.4 |
| 46 | 10 | 8 | 2 | 2 | 0 | 0 | Zr = 3 | 100 | 67.2 | 2.3 | 69.5 |
| 47 | 10 | 8 | 2 | 2 | Cs = 0.01 | V = 0.1 | Ti = 7 | 100 | 61.9 | 2.5 | 64.4 |
| 48 | 10 | 8 | 2 | 1 | Cs = 0.005 | 0 | Ti = 1 | 100 | 76.5 | 2.2 | 78.7 |

the following ranges: b=2 to 12, c=0.5 to 7, d=0.1 to 5, e=0.0005 to 0.5, f=0.01 to 2 and g=0 to 5, and the subscript h represents a positive number corresponding to the number of oxygen atoms satisfying the average valency of the respective metal atoms.

2. A process as claimed in Claim 1, wherein the contact of said feed gas with said catalyst is carried out at a temperature of from 250 to 500° C.

3. A process as claimed in either of Claims 1 and 2, wherein the contact time is in a range of from 0.3 to 20 s.

4. A process as claimed in any one of Claims 1 through 3, wherein said catalyst is combined with a carrier consisting of at least one member selected from the group consisting of silica, alumina, alumina-silica, titania, diatomaceous earth and carborundum.

5. A process as claimed in any one of Claims 1 through 4, wherein said feed gas contains at least one dilute gas selected from the group consisting of steam, nitrogen, carbon dioxide, n-butane, isobutane and propane.

6. A process as claimed in any one of Claims 1 through 5, wherein said feed gas contains 0.05 to 10 mol of steam per mole of tert.-butyl alcohol.

7. A process as claimed in any one of Claims 1 through 6, wherein the amount of said molecular oxygen in said feed gas is in a range of from 0.4 to 5 mol per mole of tert.-butyl alcohol.

8. A process as claimed in any one of Claims 1 through 7, wherein said catalyst is one prepared by uniformly mixing, in an aqueous medium, respective metal-containing compounds in the form of an oxide, salt or a mixture of said oxide and salt, by drying said mixture at a temperature not exceeding 300° C, by shaping said dried mixture, and by calcining said shaped mixture at a temperature of from 550 to 800° C.

9. A process as claimed in Claim 8, wherein said drying operation is effected in two steps, in the first step of which, said mixture is heated at a temperature of from 100 to 150° C and in the second step of which, said first dried mixture is heated at a temperature of from 150 to 300° C.

10. A process as claimed in Claim 1, wherein in the empirical formula (I), the values of said subscripts b through g are respectively in the ranges of: b=4 to 10, c=1 to 5, d=0.5 to 4, e=0.001 to 0.3, f=0.05 to 1.5 and g=0.1 to 5.

**Revendications**

1. Un procédé pour la production de méthacroléine et d'acide méthacrylique par l'oxydation catalytique de l'alcool tert.-butylique, consistant à mettre en contact un gaz d'alimentation contenant de l'alcool tert.-butylique et de l'oxygène moléculaire avec un catalyseur à température élevée, procédé qui est caractérisé en ce que ledit catalyseur consiste en une composition d'oxydes de formule empirique (I)

$$Mo_aCo_bFe_cBi_dCs_eX_fY_gO_h$$

dans laquelle Mo représente un atome de molybdène, Co représente un atome de cobalt, Fe représente un atome de fer, Bi représente un atome de bismuth, Cs représente un atome de césium, X représente au moins un atome choisi parmi les atomes de vanadium et de palladium, Y représente au moins un atome choisi parmi les atomes de titane, d'étain et de zirconium, O représente un atome d'oxygène, les indices a à g représentent

respectivement un nombre positif proportionnel au nombre des atomes de métaux respectifs, où lorsque a=12, b à g sont compris respectivement dans les gammes suivantes: b=2 à 12, c=0,5 à 7, d=0,1 à 5, e=0,0005 à 0,5, f=0,01 à 2 et g=0 à 5 et l'indice h représente un nombre positif correspondant au nombre d'atomes d'oxygène satisfaisant la valence moyenne des atomes métalliques respectifs.

2. Un procédé selon la revendication 1, dans lequel la mise en contact dudit gaz d'alimentation avec ledit catalyseur est effectuée à une température de 250 à 500° C.

3. Un procédé selon l'une des revendications 1 ou 2, dans lequel la durée de contact est dans l'intervalle de 0,3 à 20 s.

4. Un procédé selon l'une des revendications 1 à 3, dans lequel ledit catalyseur est combiné avec un support consistant en au moins une substance choisie parmi la silice, l'alumine, l'alumine-silice, l'oxyde de titane, la terre d'infusoires et le carborundum.

5. Un procédé selon l'une des revendications 1 à 4, dans lequel ledit gaz d'alimentation contient au moins un gaz dilué choisi parmi la vapeur d'eau, l'azote, le dioxyde de carbone, le n-butane, l'isobutane et le propane.

6. Un procédé selon l'une des revendications 1 à 5, dans lequel ledit gaz d'alimentation contient 0,05 à 10 mol de vapeur d'eau par mole d'alcool tert.-butylique.

7. Un procédé selon l'une des revendications 1 à 6, dans lequel la quantité dudit oxygène moléculaire dans ledit gaz d'alimentation est comprise dans l'intervalle de 0,4 à 5 mol par mole d'alcool tert.-butylique.

8. Un procédé selon l'une des revendications 1 à 7, dans lequel ledit catalyseur est un catalyseur préparé par mélange uniforme dans un milieu aqueux des composés métalliques respectifs sous la forme d'un oxyde, d'un sel ou d'un mélange dudit oxyde et dudit sel, séchage dudit mélange à une température ne dépassant pas 300° C, moulage dudit mélange séché et calcination dudit mélange moulé à une température de 550 à 800° C.

9. Un procédé selon la revendication 8, dans lequel ladite opération de séchage est effectuée en deux étapes, ledit mélange étant chauffé dans la première étape à une température de 100 à 150° C et ledit premier mélange séché étant chauffé dans la seconde étape à une température de 150 à 300° C.

10. Un procédé selon la revendication 1, dans lequel les valeurs desdits indices b à g dans la formule empirique (I) sont prises respectivement dans les gammes suivantes: b=4 à 10, c=1 à 5, d=0,5 à 4, e=0,001 à 0,3, f=0,05 à 1,5 et g=0,1 à 5.

## Patentansprüche

1. Verfahren zur Herstellung von Methacrolein und Methacrylsäure durch katalytische Oxidation von tert.-Butylalkohol, indem man ein Speisegas, welches tert.-Butylalkohol und molekularen Sauerstoff enthält, bei erhöhter Temperatur mit einem Katalysator in Kontakt bringt, dadurch gekennzeichnet, dass der Katalysator eine Oxidzusammensetzung der empirischen Formel (I):

$$Mo_aCo_bFe_cBi_dCs_eX_fY_gO_h \qquad (I)$$

enthält, in der Mo ein Molybdänatom, Co ein Kobaltatom, Fe ein Eisenatom, Bi ein Wismutatom, Cs ein Caesiumatom, X mindestens ein Mitglied aus der Gruppe von Vanadium- und Palladiumatomen, Y mindestens ein Mitglied aus der Gruppe von Titan-, Zinn- und Zirkonatomen und O ein Sauerstoffatom darstellen, während die Indices a bis g jeweils eine positive Zahl entsprechend der Anzahl der jeweiligen Metallatome bedeuten, wobei bei a=12 die Indices b bis g jeweils innerhalb der folgenden Grenzen liegen: b=2 bis 12, c=0,5 bis 7, d=0,1 bis 5, e=0,0005 bis 0,5, f=0,01 bis 2 und g= 0 bis 5, während h eine positive Zahl entsprechend der Anzahl der Sauerstoffatome repräsentiert, welche der mittleren Valenz der jeweiligen Metallatome genügt.

2. Verfahren nach Anspruch 1, bei dem der Kontakt des Speisegases mit dem Katalysator bei einer Temperatur von 250-500° C erfolgt.

3. Verfahren nach einem der Ansprüche 1 oder 2, bei dem die Kontaktzeit im Bereich von 0,3 bis 20 s liegt.

4. Verfahren nach einem der Ansprüche 1 bis 3, bei dem der Katalysator mit einem Träger kombiniert ist, bestehend aus mindestens einem Mitglied, welches ausgewählt ist aus der Gruppe von Siliciumdioxid, Aluminiumoxid, Aluminiumoxid-Siliciumdioxid, Titandioxid, Kieselgur und Carborundum.

5. Verfahren nach einem der Ansprüche 1 bis 4, bei dem das Speisegas mindestens ein verdünntes Gas enthält, welches ausgewählt ist aus der Gruppe bestehend aus Dampf, Stickstoff, Kohlendioxid, n-Butan, Isobutan und Propan.

6. Verfahren nach einem der Ansprüche 1 bis 5, bei dem das Speisegas 0,05 bis 10 mol Dampf je Mol tert.-Butylalkohol enthält.

7. Verfahren nach einem der Ansprüche 1 bis 6, bei dem die Menge des molekularen Sauerstoff in dem Speisegas im Bereich von 0,4 bis 5 mol je Mol tert.-Butylalkohol liegt.

8. Verfahren nach einem der Ansprüche 1 bis 7, bei dem der Katalysator dadurch hergestellt ist, dass die jeweiligen metallhaltigen Verbindungen in Form eines Oxids, Salzes oder einer Mischung aus diesem Oxid und dem Salz gleichmässig in einem wässrigen Medium gemisch werden, dass man diese Mischung bei einer Temperatur von nicht mehr als 300° C trocknet, die trockene Mischung zu Körpern formt und diese geformten Körper der Mischung bei einer Temperatur von 550 bis 800° C calciniert.

9. Verfahren nach Anspruch 8, bei dem die Trocknung in zwei Stufen durchgeführt wird, und zwar einer ersten Stufe, bei der die Mischung auf eine Temperatur von 100 bis 150° C erhitzt wird,

und einer zweiten Stufe, bei der diese zuerst getrocknete Mischung auf eine Temperatur von 150 bis 300° C erhitzt wird.

10. Verfahren nach Anspruch 1, bei dem in der empirischen Formel (I) die Werte der indices b bis g jeweils in den Bereichen liegen von: b=4 bis 10, c=1 bis 5, d=0,5 bis 4, e=0,001 bis 0,3, f=0,05 bis 1,5 und g=0,1 bis 5.